Europäisches Patentamt

**European Patent Office**

**Office européen des brevets**

(11) Numéro de publication: **0 359 625 B1**

(12) FASCICULE DE BREVET EUROPEEN

(45) Date de publication de fascicule du brevet: **15.12.93** (51) Int. Cl.5: **A61K 9/70**, A61L 15/16

(21) Numéro de dépôt: **89402421.5**

(22) Date de dépôt: **06.09.89**

(54) **Dispositif auto-adhésif d'administration d'un principe actif par voie percutanée.**

(30) Priorité: **07.09.88 FR 8811685**

(43) Date de publication de la demande:
**21.03.90 Bulletin 90/12**

(45) Mention de la délivrance du brevet:
**15.12.93 Bulletin 93/50**

(84) Etats contractants désignés:
**AT BE CH DE ES FR GB GR IT LI LU NL SE**

(56) Documents cités:
**EP-A- 0 159 168**
**EP-A- 0 272 987**
**FR-A- 2 612 785**
**US-A- 4 568 343**

(73) Titulaire: **LABORATOIRES D'HYGIENE ET DE DIETETIQUE**
**38 avenue Hoche**
**F-75008 Paris(FR)**

(72) Inventeur: **Guillemet, Alain**
**12, rue Martin de Noinville**
**F-21000 Dijon(FR)**
Inventeur: **Teillaud, Eric**
**1, allée Roger Bernard**
**F-21240 Talant(FR)**
Inventeur: **Reginault, Philippe**
**13, rue de Provence**
**F-21121 Fontaine les Dijon(FR)**
Inventeur: **Bevan, Bruno**
**32 cours du Parc**
**F-21000 Dijon(FR)**

(74) Mandataire: **Clisci, Serge et al**
**S.A. FEDIT-LORIOT**
**CONSEILS EN PROPRIETE INDUSTRIELLE**
**38, avenue Hoche**
**F-75008 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne une nouvelle matrice pour l'administration d'un principe actif par voie percutanée.

Par matrice, on entend ici la composition chimique des ingrédients permettant l'administration percutanée d'un principe actif, en vue d'un effet thérapeutique local, local profond ou systémique, et par dispositif, on entend l'ensemble constitué par la matrice et son support.

Il a déjà été proposé de nombreux systèmes pour l'administration d'un principe actif par voie percutanée. On connaît en particulier des systèmes destinés à administrer de la trinitroglycérine par voie percutanée pour le traitement de l'angine de poitrine. Ces systèmes, qui sont constitués d'un support sur lequel est déposé un réservoir ou une matrice contenant le principe actif, présentent l'inconvénient d'une adhésivité à la peau qui décroît rapidement au cours du temps. En effet, dans le cas d'un réservoir, le principe actif est dissous dans un solvant servant de vecteur de transport du principe actif à travers une membrane microporeuse vers la peau. Dans le cas d'une matrice, le principe actif contenu dans un réseau polymérique est également dissous dans un solvant servant de vecteur de transport. Le réservoir ou la matrice étant maintenus sur le peau par un adhésif classique, du type masse acrylique, le solvant dissout partiellement certains composants de l'adhésif qui perd ainsi rapidement son efficacité.

Pour remédier à cet inconvénient, il a déjà été proposé, dans EP-A-0159168, une solution telle que la matrice contenant le principe actif a des propriétés d'auto-adhérence à la peau. Cette matrice, contenant un principe actif, est constituée d'une protéine soluble dans l'eau, d'un alcool polyhydrique, d'un agent tackifiant et d'une substance oléagineuse. Plus particulièrement la protéine soluble dans l'eau peut être naturelle ou synthétique, animale ou végétale, comme par exemple la gélatine, le collagène, la caséine ou la glu, dans des proportions de 5 à 50 % ; l'alcool polyhydrique peut être un glycol, comme par exemple l'éthylène glycol, le propylène glycol, le butylène glycol ou le polyéthylène glycol, un triol ou un polyol, dans des proportions de 5 à 50 % ; l'agent tackifiant peut être de la cellulose ou un dérivé, comme par exemple la méthylcellulose, l'éthylcellulose, la propylcellulose, la méthylpropylcellulose, l'hydroxyméthylcellulose, l'hydroxyéthylcellulose, l'hydroxypropylcellulose et la carboxyméthylcellulose, un polysaccharide, l'alcool polyvinylique ou la polyvinylpyrrolidone, dans des proportions de 0,1 à 15 % ; et la substance oléagineuse peut être un ester d'acide gras, la paraffine, la lanoline, un alcool aliphatique supérieur, comme par exemple les alcools octyldodécylique, palmitique, stéarique et myristique, ou l'huile de silicone, dans des proportions de 0,1 à 25 %.

Un exemple de matrice contenant du mono-laurate de glycérol et tant que promoteur de la perméation est donné par le brevet US-A-4746515.

Dans ce brevet le mono-laurate de glycérol constitue 25 % de la matrice contenant 36 % d'EVA 40 (c'est-à-dire ayant 40 % de motifs acétate de vinyle).

On sait que l'on a déjà préconisé dans le passé, dans des dispositifs pour absorption percutanée d'un médicament, des matrices comprenant un EVA et un promoteur d'absorption du type ester d'acide gras. Ainsi on connait de EP-A-0 272 987 une composition à base d'EVA et de mono-laurate de propylène glycol, et de US-A-4 568 343 une composition à base d'EVA et de mono-laurate de polyéthylène glycol. De plus, selon EP-A-0 272 987 (voir page 4, lignes 4-7) et US-A-4 568 343 (voir exemple III, colonne 5) il est recommandé, pour obtenir les propriétés mécaniques souhaitées, un dispositif comprenant plusieurs couches de matrice.

On propose à présent une nouvelle solution technique faisant appel à une matrice auto-adhésive différente de l'art antérieur constitué par EP-A-0 159 158 par la nature du constituant principal, par les proportions des autres constituants, ainsi que par la facilité de mise en oeuvre industrielle, différente de l'art antérieur constitué par US-A-4 746 515 par le fait que les constituants de la phase liquide de la matrice selon l'invention sont en quantité telle qu'ils ne sont pas utilisés en tant que promoteurs de la perméation mais en tant que solvants du principe actif, et différente de l'art antérieur constitué par EP-A-0 272 987 et US-A-4 568 343 par le choix des composants b) et c) ci-après et le fait que la matrice selon l'invention constitue une seule couche au lieu de la configuration à plusieurs couches recommandée par lesdits EP-A-0 272 987 et US-A-4 568 343.

La matrice auto-adhésive pour l'administration d'un principe actif par voie percutanée selon l'invention est caractérisée en ce qu'elle comprend :

a) 30 à 50 parties en poids de copolymère d'éthylène et d'acétate de vinyle,
b) 20 à 45 parties en poids d'alcool aliphatique supérieur choisi parmi les mono-alcools gras en $C_{12}-C_{20}$,
c) 5 à 20 parties en poids de dérivé de cellulose,
d) 1 à 20 parties en poids d'un ester d'acide gras et d'alcool polyhydrique, et
e) 0,1 à 20 parties en poids de principe actif administrable par voie percutanée.

De façon avantageuse, on utilisera un copolymère d'éthylène et d'acétate de vinyle (EVA) ayant une teneur en motifs acétate de vinyle comprise entre 35 et 55 % en poids, de préférence de l'ordre de 45 % en poids, par rapport au poids du copolymère d'éthylène/acétate de vinyle.

Par alcool aliphatique supérieur, on entend ici les mono-alcools saturés ou insaturés ayant 12 à 20 atomes de carbone comme par exemple le 2-octyl-1-dodécanol, l'alcool palmitique, l'alcool stéarique ou l'alcool myristique.

Par dérivé de cellulose, on entend ici les alkylcelluloses, comme par exemple la méthylcellulose, l'éthylcellulose, la propylcellulose ou la méthylpropylcellulose et les hydroxyalkylcelluloses, comme par exemple l'hydroxyméthylcellulose, l'hydroxyéthylcellulose ou l'hydroxypropylcellulose.

Par ester d'acide gras et d'alcool polyhydrique, on entend ici les esters obtenus entre un alcool polyhydrique, notamment le glycérol et les glycols, en particulier les alkylène glycols, comme par exemple l'éthylène glycol, le propylène glycol, le dipropylène glycol, le butylène glycol, le triéthylène glycol et le diéthylène glycol ou le polyéthylène glycol et le polypropylène glycol, et un acide comportant un nombre total d'atomes de carbone de 8 à 18, comme par exemple l'acide pélargonique, l'acide stéarique, l'acide isostéarique, l'acide caprique, l'acide caprylique, l'acide palmitique, l'acide laurique, l'acide myristique ou l'acide oléique.

Par principe actif, on entend ici tout produit percutanéable solide ou liquide qui est soluble, au moins partiellement, dans la phase constituée par les moyens b) et d). On préférera les principes actifs anti-inflammatoires non stéroïdiens comme par exemple l'ibuprofène, le kétoprofène, l'acide niflumique ou l'acide méfénamique, les antagonistes calciques du type dihydropyridine comme par exemple la nifédipine ou la nicardipine, les $\beta$-bloquants comme par exemple le timolol ou le propranolol et les $\beta$-stimulants comme par exemple le procatérol ou le salbutamol.

Pratiquement, on préférera une teneur en principe actif telle que ledit principe actif sature la phase liquide constituée par les moyens b) et d) et on préférera les formulations telles que le rapport de la phase solvant [constituée par les moyens b) et d)] à la phase polymère [constituée par les moyens a) et c)] soit voisin de l'unité.

Le support recevant la matrice pourra être tout support souple, imperméable aux constituants de la matrice et d'épaisseur comprise entre 50 et 1000 micromètres. On préférera un support polymérique tel que par exemple (i) le polyéthylène, le polypropylène, les polyesters, (ii) les mousses, notamment microcellulaires, de polyéthylène connues notamment dans le domaine des pansements, et (iii) les copolymères d'au moins deux monomères choisis parmi l'éthylène, le propylène, les acrylates et méthacrylates d'alkyle en $C_1$-$C_3$.

De façon pratique, la matrice pourra être recouverte d'une pellicule de protection, pelable avant utilisation du dispositif, lequel pourra être lui-même emballé dans une protection étanche comme par exemple les complexes polyéthylène-aluminium.

Le dispositif auto-adhésif d'administration d'un principe actif par voie percutanée selon l'invention, peu collant (ou poisseux, en anglais "tacky") immédiatement au contact de la peau, est doué d'un pouvoir adhésif progressif se renforçant dès quelques minutes après mise en place sur la peau d'un sujet. Le dispositif ou la matrice peut être facilement enlevé sans desquamation de la peau puis reposé en gardant un pouvoir adhésif identique. Ceci est particulièrement avantageux pour un tel dispositif devant être gardé plusieurs jours par un sujet qui peut ainsi enlever momentanément ledit dispositif, par exemple lors de la prise d'un bain.

Le dispositif selon l'invention présente encore l'avantage d'être réduit à une surface et une épaisseur minimales. De par son propre pouvoir adhésif, il n'est pas nécessaire de border la partie active du dispositif par une masse adhésive. Toute la surface du dispositif est à la fois active et adhésive. Des épaisseurs de quelques centaines de micromètres de matrice (150 à 250 micromètres correspondant à un grammage de 100 à 300 g/cm²) suffiront pour délivrer à travers la peau pendant plusieurs jours la quantité nécessaire de principe actif.

Suivant l'invention, on préconise un procédé pour la préparation d'une matrice auto-adhésive pour l'administration percutanée d'un principe actif qui comprend les étapes suivantes :

1) on mélange sous agitation le moyen a) et une fraction du moyen b) à une température supérieure ou égale à 110°C et on homogénéise le mélange pendant environ 0,5 heure,

2) on incorpore, sous agitation et par petites fractions, à une température supérieure ou égale à 110°C le moyen c) dans le mélange résultant du stade 1) puis homogénéise,

3) on incorpore le reste du moyen b), sous agitation à une température supérieure ou égale à 110°C, au mélange résultant du stade 2),

4) on homogénéise le mélange résultant ainsi obtenu à une température supérieure ou égale à 110°C puis le laisse reposer pendant au moins 8 heures,

5) on chauffe le mélange résultant ainsi obtenu à une température de 50-70°C, de préférence à 60°C, pendant au moins 0,25 h, puis incorpore à cette température le moyen d) et le principe actif dans un solvant dudit principe actif, par exemple l'éthanol, ledit solvant représentant 30 à 100 %, de préférence environ 80 %, en volume par rapport au poids total des moyens a), b), c), d) et e),

6) on homogénéise le mélange résultant pendant au moins 0,5 h à une température de 50-70°C, de préférence à 60°C,

7) on dépose le mélange résultant ainsi homogénéisé sur un support temporaire, notamment du papier siliconé, à une température de l'ordre de 50-70°C à raison de 100 à 300 g/m², 

8) on chauffe l'ensemble comprenant ledit support temporaire et la matrice à une température de l'ordre de 70-90°C pour évaporer le solvant du principe actif jusqu'à obtenir un taux résiduel inférieur à 5 %,

9) on transfère la matrice sèche résultante sur un support approprié.

La production industrielle du dispositif selon l'invention est facilitée par le fait que la matrice chargée en principe actif est malléable et également enductible sur un support par la technique dite de "voie fondue", c'est-à-dire par fusion en l'absence de solvant. Quelle que soit la technique d'enduction employée (en phase solvant ou "voie fondue"), on peut ainsi enduire de grandes surfaces puis découper le dispositif à la dimension voulue, calculée en fonction de la quantité de principe actif présent par unité de surface et de la quantité de principe actif à délivrer au sujet pendant un temps déterminé.

Cette technique simple de fabrication par découpage à une surface variable est particulièrement intéressante pour commercialiser des dispositifs de différentes dimensions, donc permettant des apports différents en principe actif. On sait en effet que pour certains principes actifs, notamment les β-bloquants et les β-stimulants, la posologie journalière doit être adaptée au sujet en fonction des résultats obtenus. On pourra, en particulier, avec le dispositif selon l'invention, augmenter ou diminuer progressivement la dose en utilisant des dispositifs de surface croissante, ou décroissante, jusqu'à obtenir l'effet thérapeutique exactement souhaité.

Différents dispositifs selon l'invention ont été réalisés et on a étudié ex vivo les cinétiques de perméation sur un disque de 3,14 cm² de peau abdominale de souris "nude" en cellule statique en verre ayant un compartiment receveur d'un volume de 31 ml, agitée au moyen d'un système magnétique et immergée dans un bain thermostaté à 37°C. Dans les exemples de préparation qui suivent on a donné les résultats moyens obtenus à partir de 3 échantillons identiques de chacune des préparations.

## PREPARATION I

### Example 1

On introduit dans un malaxeur de 5 litres 1375 g de LEVAPREN 450 P$^R$ (EVA ayant une teneur en motifs acétate de vinyle de 45 % commercialisé par la Société BAYER) et 840 g d'EUTANOL G$^R$ (2-octyl-dodécanol commercialisé par la Société HENKEL). On chauffe à 140°C pendant 0,5 heure et on additionne au mélange, par petites fractions, 305 g d'ETHOCEL 20$^R$ (éthylcellulose de viscosité égale à 2x10$^{-2}$ Pa.s commercialisé par la Société DOW CHEMICAL). Après homogénéisation du milieu, on ajoute 420 g d'EUTANOL G$^R$. On homogénéise l'ensemble pendant 0,5 heure et on laisse reposer pendant 24 heures. On porte ensuite la masse à 60°C pendant 0,5 heure et on additionne, par fractions, une solution de 350 g de LAUROGLYCOL$^R$ (mélange de mono et di-ester de propylène glycol et d'acide laurique commercialisé par la Société GATTEFOSSE) et 210 g d'acide niflumique dans 1910 g d'éthanol anhydre. On homogénéise l'ensemble pendant 1 heure à 60°C. On enduit cette masse sur un papier siliconé de 105 mm de large à une température de 60°C à raison de (148 ± 5) g/m². Après passage du papier siliconé enduit à 80°C pour évaporer l'éthanol jusqu'à un taux inférieur à 3,5 %, on transfère la matrice sur un support polyéthylène.

- Caractéristiques de la matrice :

- moyen a) : 39,3 %
- moyen b) : 36 %
- moyen c) : 8,7 %
- moyen d) : 10 %
- moyen e) : 6 %
- dose de principe actif : 888 $\mu$g/cm²

- Résultats moyens obtenus en cinétique de perméation ex vivo :

- éluant : sérum physiologique (NaCl à 0,9 %)-/PEG 400 (80/20, v/v)
- quantité de principe actif absorbé en 24 heures : 29,2 %
- flux moyen : 11,6 $\mu$g/cm²/h ± 12,3 %

## PREPARATION II

### Example 2

On procède de manière identique à la préparation I en remplaçant le LAUROGLYCOL$^R$ par la même quantité de LABRASOL$^R$ (mélange d'esters caprylo-capriques du glycérol et du PEG 400 commercialisé par la Société GATTEFOSSE) et en enduisant à raison de (138 ± 5) g/m$^2$.

- Caractéristiques de la matrice :

- moyen a) : 39,3 %
- moyen b) : 36 %
- moyen c) : 8,7 %
- moyen d) : 10 %
- moyen e) : 6 %
- dose de principe actif : 828 $\mu$g/cm$^2$

- Résultats moyens obtenus en cinétique de perméation ex vivo :

- éluant : sérum physiologique (NaCl à 0,9 %)-/PEG 400 (80/20, v/v)
- quantité de principe actif absorbé en 24 heures : 32,6 %
- flux moyen : 12,1 $\mu$g/cm$^2$/h ± 6,8 %

## PREPARATION III

### Example 3

De façon analogue à la préparation I, on mélange 1505 g de LEVAPREN 450P$^R$, 925 g d'EUTANOL G$^R$ et 330 g d'ETHOCEL 20$^R$ à 140°C puis on ajoute 460 g d'EUTANOL G$^R$. On additionne ensuite, à 60°C, 105 g de MIGLYOL 840$^R$ -(mélange de diesters du propylène glycol et des acides capriques et capryliques commercialisé par la Société DYNAMIT NOBEL) et 175 g d'ibuprofène dans 1910 g d'éthanol anhydre. On procède ensuite de manière identique à la préparation I en enduisant à raison de (181 ± 5) g/m$^2$.

- Caractéristiques de la matrice :

- moyen a) : 43 %
- moyen b) : 39,5 %
- moyen c) : 9,5 %
- moyen d) : 3 %
- moyen e) : 5 %
- dose de principe actif : 905 $\mu$g/cm$^2$

- Résultats moyens obtenus en cinétique de perméation ex vivo :

- éluant : tampon phosphate pH = 7,4
- quantité de principe actif absorbé en 24 heures : 56 %
- flux moyen : 22,8 $\mu$g/cm$^2$/h ± 19 %

## PREPARATION IV

### Exemple 4

On procède de manière identique à la préparation III en remplaçant le MIGLYOL 840$^R$ par la même quantité de D.P.P.G.$^R$ (dipélargonate de propylène glycol commercialisé par la Société GATTEFOSSE) et en enduisant à raison de (183 ± 5) g/m$^2$.

- Caractéristiques de la matrice :

- moyen a) : 43 %
- moyen b) : 39,5 %
- moyen c) : 9,5 %
- moyen d) : 3 %
- moyen e) : 5 %
- dose de principe actif : 915 $\mu$g/cm$^2$

- Résultats moyens obtenus en cinétique de perméation ex vivo :

- éluant : tampon phosphate pH = 7,4
- quantité de principe actif absorbé en 24 heures : 62 %
- flux moyen : 25,4 $\mu$g/cm$^2$/h ± 19 %

## PREPARATION V

### Exemple 5 :

De façon analogue à la préparation I, on mélange 1425 g de LEVAPREN 450P$^R$, 870 g d'EUTANOL G$^R$ et 315 g d'ETHOCEL 20$^R$ à 140°C puis on ajoute 435 g d'EUTANOL G$^R$. On additionne ensuite, à 60°C, 105 g de D.P.P.G.$^R$ et 350 g d'ibuprofène dans 1910 g d'éthanol anhydre. On procède ensuite de manière identique à la préparation I en enduisant à raison de (164 ± 5) g/m$^2$.

- Caractéristiques de la matrice :

- moyen a) : 40,7 %
- moyen b) : 37,3 %
- moyen c) : 9 %
- moyen d) : 3 %
- moyen e) : 10 %
- dose de principe actif : 1640 $\mu$g/cm$^2$

- Résultats moyens obtenus en cinétique de perméation ex vivo :

- éluant : tampon phosphate pH = 7,4
- quantité de principe actif absorbé en 24 heures : 50 %
- flux moyen : 35,7 $\mu$g/cm$^2$/h ± 5 %

## PREPARATION VI

### Exemple 6

De façon analogue à la préparation I, on mélange 1505 g de LEVAPREN 450P[R], 925 g d'EUTANOL G[R] et 330 g d'ETHOCEL 20[R] à 140°C puis on ajoute 460 g d'EUTANOL G[R]. On additionne ensuite, sous éclairage inactinique et à 60°C, 105 g de LAUROGLYCOL[R] et 175 g de nifédipine dans 1910 g d'éthanol anhydre. On procède ensuite de manière identique à la préparation I en enduisant à raison de (125 ± 5) g/m².

- Caractéristiques de la matrice :

- moyen a) : 43 %
- moyen b) : 39,5 %
- moyen c) : 9,5 %
- moyen d) : 2,9 %
- moyen e) : 5 %
- dose de principe actif : 625 $\mu$g/cm²

- Résultats moyens obtenus en cinétique de perméation ex vivo :

- éluant : sérum physiologique (NaCl à 0,9 %)-/PEG 400 (70/30, v/v)
- quantité de principe actif absorbé en 24 heures : 6,4 %
- flux moyen : 1,99 $\mu$g/cm²/h ± 50 %

## PREPARATION VII

### Exemple 7

De façon analogue à la préparation I, on mélange 1390 g de LEVAPREN 450P[R], 850 g d'EUTANOL G[R] et 310 g d'ETHOCEL 20[R] à 140°C puis on ajoute 425 g d'EUTANOL G[R]. On additionne ensuite, à 60°C, 350 g d'HYDROPHILOL ISOSTEARIQUE[R] (isostéarate de propylène glycol commercialisé par la Société GATTEFOSSE) et 175 g de salbutamol dans 1910 g d'éthanol anhydre. On procède ensuite de manière identique à la préparation I en enduisant à raison de (102 ± 5) g/m².

- Caractéristiques de la matrice :

- moyen a) : 39,7 %
- moyen b) : 36,5 %
- moyen c) : 8,8 %
- moyen d) : 10 %
- moyen e) : 5 %
- dose de principe actif : 510 $\mu$g/cm²

- Résultats moyens obtenus en cinétique de perméation ex vivo :

- éluant : sérum physiologique (NaCl à 0,9 %)-/PEG 400 (70/30, v/v)
- quantité de principe actif absorbé en 24 heures : 83,5 %
- flux moyen : 18,44 $\mu$g/cm²/h ± 10,6 %

## PREPARATION VIII

### Exemple 8

On procède de manière identique à la préparation VII en remplaçant l'HYDROPHILOL ISOSTEARIQUE[R] par la même quantité de LAUROGLYCOL[R] et en enduisant à raison de (106 ± 5) g/m².

- Caractéristiques de la matrice :

- moyen a) : 39,7 %
- moyen b) : 36,5 %
- moyen c) : 8,8 %
- moyen d) : 10 %
- moyen e) : 5 %
- dose de principe actif : 530 $\mu$g/cm²

- Résultats moyens obtenus en cinétique de perméation ex vivo :

- éluant : sérum physiologique (NaCl à 0,9 %)-/PEG 400 (70/30, v/v)
- quantité de principe actif absorbé en 24 heures : 93,9 %
- flux moyen : 20,35 $\mu$g/cm²/h ± 4,9 %

## PREPARATION IX

### Exemple 9

De façon analogue à la préparation I, on mélange 1309 g de LEVAPREN 450P[R], 800 g d'EUTANOL G[R] et 290 g d'ETHOCEL 20[R] à 140°C puis on ajoute 400 g d'EUTANOL G[R]. On additionne ensuite, à 60°C, 350 g de MIGLYOL 840[R] et 350 g de timolol base dans 1910 g d'éthanol anhydre. On procède ensuite de manière identique à la préparation I en enduisant à raison de (283 ± 5) g/m².

- Caractéristiques de la matrice :

- moyen a) : 37,4 %
- moyen b) : 34,3 %
- moyen c) : 8,3 %
- moyen d) : 10 %
- moyen e) : 10 %
- dose de principe actif : 2830 $\mu$g/cm²

- Résultats moyens obtenus en cinétique de perméation ex vivo :

- éluant : tampon phosphate pH = 7,4
- quantité de principe actif absorbé en 24 heures : 73,6 %
- flux moyen : 77,2 $\mu$g/cm$^2$/h ± 11 %

## PREPARATION X

### Exemple 10

On procède de manière identique à la préparation IX en remplaçant le MIGLYOL 840$^R$ par la même quantité d'HYDROPHILOL ISOSTEARIQUE$^R$ et en enduisant à raison de (273 ± 5) g/m$^2$.

- Caractéristiques de la matrice :

- moyen a) : 37,4 %
- moyen b) : 34,3 %
- moyen c) : 8,3 %
- moyen d) : 10 %
- moyen e) : 10 %
- dose de principe actif : 2725 $\mu$g/cm$^2$

- Résultats moyens obtenus en cinétique de perméation ex vivo :

- éluant : tampon phosphate pH = 7,4
- quantité de principe actif absorbé en 24 heures : 74 %
- flux moyen : 74,9 $\mu$g/cm$^2$/h ± 3,0 %

## PREPARATION XI

### Exemple 11

On procède de manière identique à la préparation IX en remplaçant le MIGLYOL 840$^R$ par la même quantité de D.P.P.G.$^R$ et en enduisant à raison de (278 ± 5) g/m$^2$.

- Caractéristiques de la matrice :

- moyen a) : 37,4 %
- moyen b) : 34,3 %
- moyen c) : 8,3 %
- moyen d) : 10 %
- moyen e) : 10 %
- dose de principe actif : 2780 $\mu$g/cm$^2$

- Résultats moyens obtenus en cinétique de perméation ex vivo :

- éluant : tampon phosphate pH = 7,4
- quantité de principe actif absorbé en 24 heures : 73,3 %

- flux moyen : 73,5 $\mu$g/cm$^2$/h ± 1 %

**Revendications**

**Revendications pour les Etats contractants suivants : AT, BE, CH, DE, FR, GB, IT, LI, LU, NL, SE**

1. Matrice auto-adhésive pour l'administration d'un principe actif par voie percutanée, caractérisée en ce qu'elle comprend :

a) 30 à 50 parties en poids de copolymère d'éthylène et d'acétate de vinyle,

b) 20 à 45 parties en poids d'alcool aliphatique supérieur choisi parmi les mono-alcools gras en C$_{12}$-C$_{20}$,

c) 5 à 20 parties en poids de dérivé de cellulose,

d) 1 à 20 parties en poids d'un ester d'acide gras et d'alcool polyhydrique, et

e) 0,1 à 20 parties en poids de principe actif administrable par voie percutanée.

2. Matrice suivant la revendication 1 caractérisée en ce que le copolymère d'éthylène et d'acétate de vinyle a une teneur en motifs acétate de vinyle comprise entre 35 et 55 % en poids par rapport au poids dudit copolymère.

3. Matrice suivant la revendication 2, caractérisée en ce que le copolymère d'éthylène et d'acétate de vinyle a une teneur en motifs acétate de vinyle de 45 % en poids par rapport au poids dudit copolymère.

4. Matrice suivant la revendication 1, caractérisée en ce que l'alcool aliphatique supérieur en C$_{12}$-C$_{20}$ est choisi parmi l'ensemble comprenant les mono-alcools saturés et insaturés.

5. Matrice suivant la revendication 1, caractérisée en ce que le dérivé de cellulose est choisi parmi l'ensemble comprenant les alkylcelluloses et hydroxyalkylcelluloses, notamment les méthylcellulose, éthylcellulose, propylcellulose, méthylpropylcellulose, hydroxyméthylcellulose, hydroxyéthylcellulose et hydroxypropylcellulose.

6. Matrice suivant la revendication 1, caractérisée en ce que l'ester d'acide gras et d'alcool polyhydrique est choisi parmi l'ensemble comprenant les esters obtenus entre le glycérol ou un glycol et un acide comportant un nombre total d'atomes de carbone de 8 à 18.

7. Matrice suivant la revendication 6, caractérisée en ce que le glycol est choisi parmi l'ensemble comprenant les alkylène glycols, notamment les éthylène glycol, propylène glycol, dipropylène glycol butylène glycol, triéthylène glycol, diéthylène glycol, polyéthylène glycol, polypropylène glycol.

8. Matrice suivant la revendication 6, caractérisée en ce que l'acide gras est choisi parmi l'ensemble comprenant les acides pélargonique, stéarique, isostéarique, caprique, caprylique, palmitique, laurique, myristique, oléique.

9. Matrice suivant la revendication 1, caractérisée en ce que le principe actif administrable par voie percutanée est un agent anti-inflammatoire non stéroïdien, notamment l'ibuprofène, le kétoprofène, l'acide niflumique ou l'acide méfénamique.

10. Matrice suivant la revendication 1, caractérisée en ce que le principe actif administrable par voie percutanée est un agent antagoniste calcique du type dihydropyridine, notamment la nifédipine ou la nicardipine.

11. Matrice suivant la revendication 1, caractérisée en ce que le principe actif administrable par voie percutanée est un agent $\beta$-bloquant, notamment le timolol ou le propranolol.

12. Matrice suivant la revendication 1, caractérisée en ce que le principe actif administrable par voie percutanée est un agent $\beta$-stimulant, notamment le procatérol ou le salbutamol.

13. Matrice suivant la revendication 1, caractérisée en ce que le rapport pondéral a) + c) / b) + d) est de l'ordre de 1.

14. Procédé de préparation d'une matrice suivant la revendications 1, caractérisé en ce que :
1) on mélange sous agitation le moyen a) et une fraction du moyen b) à une température supérieure ou égale à 110°C et on homogénéise le mélange pendant 0,5 heure,
2) on incorpore, sous agitation et par petites fractions, à une température supérieure ou égale à 110°C le moyen c) dans le mélange résultant du stade 1) puis homogénéise,
3) on incorpore le reste du moyen b), sous agitation à une température supérieure ou égale à 110°C, au mélange résultant du stade 2),
4) on homogénéise le mélange résultant ainsi obtenu à une température supérieure ou égale à 110°C puis le laisse reposer pendant au moins 8 heures,
5) on chauffe le mélange résultant ainsi obtenu à une température de 50-70°C, pendant au moins 0,25 h, puis incorpore à cette température le moyen d) et le principe actif dans un solvant dudit principe actif, ledit solvant représentant 30 à 100 % en volume par rapport au poids total des moyens a), b), c), d) et e),
6) on homogénéise le mélange résultant pendant au moins 0,5 h à une température de 50-70°C,
7) or dépose le mélange résultant ainsi homogénéisé sur un support temporaire, notamment du papier siliconé, à une température de 50-70°C à raison de 100 à 300 g/m$^2$,
8) on chauffe l'ensemble comprenant ledit support temporaire et la matrice à une température de 70-90°C pour évaporer le solvant du principe actif jusqu'à obtenir un taux résiduel inférieur à 5 %,
9) on transfère la matrice sèche résultante sur un support approprié.

**Revendications pour les Etats contractants suivants : ES, GR**

1. Procédé de préparation d'une matrice autoadhésive pour l'administration d'un principe actif par voie percutanée et comprenant :
a) 30 à 50 parties en poids de copolymère d'éthylène et d'acétate de vinyle,
b) 20 à 45 parties en poids d'alcool aliphatique supérieur choisi parmi les mono-alcools gras en $C_{12}$-$C_{20}$,
c) 5 à 20 parties en poids de dérivé de cellulose,
d) 1 à 20 parties en poids d'un ester d'acide gras et d'alcool polyhydrique, et
e) 0,1 à 20 parties en poids de principe actif administrable par voie percutanée,
ledit procédé étant caractérisé en ce que :
1) on mélange sous agitation le moyen a) et une fraction du moyen b) à une température supérieure ou égale à 110°C et on homogénéise le mélange pendant 0,5 heure,
2) on incorpore, sous agitation et par petites fractions, à une température supérieure ou égale à 110°C le moyen c) dans le mélange résultant du stade 1) puis homogénéise,
3) on incorpore le reste du moyen b), sous agitation à une température supérieure ou égale à 110°C, au mélange résultant du stade 2),
4) on homogénéise le mélange résultant ainsi obtenu à une température supérieure ou égale à 110°C puis le laisse reposer pendant au moins 8 heures,
5) on chauffe le mélange résultant ainsi obtenu à une température de 50-70°C, pendant au moins 0,25 h, puis incorpore à cette température le moyen b) et le principe actif dans un solvant dudit principe actif, ledit solvant représentant 30 à 100 % en volume par rapport au poids total des moyens a), b), c), d) et e),

6) on homogénéise le mélange résultant pendant au moins 0,5 h à une température de 50-70°C,

7) on dépose le mélange résultant ainsi homogénéisé sur un support temporaire à une température de 50-70°C à raison de 100 à 300 g/m², 

8) on chauffe l'ensemble comprenant ledit support temporaire et la matrice à une température de 70-90°C pour évaporer le solvant du principe actif jusqu'à obtenir un taux résiduel inférieur à 5 %,

9) on transfère la matrice sèche résultante sur un support approprié.

2. Procédé selon la revendication 1, caractérisé en ce que le copolymère d'éthylène et d'acétate de vinyle a une teneur en motifs acétate de vinyle comprise entre 35 et 55 % en poids par rapport au poids dudit copolymère.

3. Procédé selon la revendication 2, caractérisé en ce que le copolymère d'éthylène et d'acétate de vinyle a une teneur en motifs acétate de vinyle de 45 % en poids par rapport au poids dudit copolymère.

4. Procédé selon la revendication 1, caractérisé en ce que l'alcool aliphatique supérieur est choisi parmi l'ensemble comprenant les mono-alcools en $C_{12}$-$C_{20}$ saturés et insaturés.

5. Procédé selon la revendication 1, caractérisé en ce que le dérivé de cellulose est choisi parmi l'ensemble comprenant les alkylcelluloses et hydroxyalkylcelluloses, notamment les méthylcellulose, éthylcellulose, propylcellulose, méthylpropylcellulose, hydroxyméthylcellulose, hydroxyéthylcellulose et hydroxypropylcellulose.

6. Procédé selon la revendication 1, caractérisé en ce que l'ester d'acide gras et d'alcool polyhydrique est choisi parmi l'ensemble comprenant les esters obtenus entre le glycérol ou un glycol et un acide comportant un nombre total d'atomes de carbone de 8 à 18.

7. Procédé selon la revendication 6, caractérisé en ce que le glycol est choisi parmi l'ensemble comprenant les alkylène glycols, notamment les éthylène glycol, propylène glycol, dipropylène glycol, butylène glycol, triéthylène glycol, diéthylène glycol, polyéthylène glycol, polypropylène glycol.

8. Procédé selon la revendication 6, caractérisé en ce que l'acide gras est choisi parmi l'ensemble comprenant les acides pélargonique, stéarique, isostéarique, caprique, caprylique, palmitique, laurique, myristique, oléique.

9. Procédé selon la revendication 1, caractérisé en ce que le principe actif administrable par voie percutanée est un agent antiinflammatoire non stéroidien, notamment l'ibuprofène, kétoprofène, l'acide niflumique ou l'acide méfénamique.

10. Procédé selon la revendication 1, caractérisé en ce que le principe actif administrable par voie percutanée est un agent antagoniste calcique du type dihydropyridine, notamment la nifédipine ou la nicardipine.

11. Procédé selon la revendication 1, caractérisé en ce que le principe actif administrable par voie percutanée est un agent bêta-bloquant, notamment le timolol ou le propranolol.

12. Procédé selon la revendication 1, caractérisé en ce que le principe actif administrable par voie percutanée est un agent bêta-stimulant, notamment le procatérol ou le salbutamol.

13. Procédé selon la revendication 1, caractérisé en ce que le rapport pondéral a) + c) / b) + d) est de l'ordre de 1.

**Claims**

**Claims for the following Contracting States : AT, BE, CH, DE, FR, GB, IT, LI, LU, NL, SE**

1. A self-adhesive matrix for the percutaneous administering of a pharmaceutical active ingredient comprising

a) 30 to 50 parts by weight of an ethylene-vinyl acetate copolymer material,

b) 20 to 45 parts by weight of a higher aliphatic monoalcohol compound, selected from the $C_{12}$-$C_{20}$ fatty monoalcohol,

c) 5 to 20 parts by weight of a cellulose derivative material,

d) 1 to 20 parts by weight of an ester compound of a polyhydric alcohol with a fatty aliphatic acid, and

e) 0.1 to 20 parts by weight of an active ingredient which can be administered percutaneously.

2. A matrix according to claim 1, wherein the ethylene/vinyl acetate copolymer material has a content of vinyl acetate units of between 35 to 55 % by weight, relative to the weight of said copolymer material.

3. A matrix according to claim 1 wherein the ethylene/vinyl acetate copolymer material has a content of vinyl acetate units of 45 % by weight, relative to the weight of said copolymer material.

4. A matrix according to claim 1 wherein the $C_{12}$-$C_{20}$ higher aliphatic monoalcohol compound is selected from the group consisting of saturated and unsaturated monoalcohols.

5. A matrix according to claim 1 wherein the cellulose derivative material is selected from the group consisting of alkyl celluloses and hydroxyalkyl celluloses, in particular methyl cellulose, ethyl cellulose, propyl cellulose, methylpropyl cellulose, hydroxymethyl cellulose, hydroxyethyl cellulose and hydroxypropyl cellulose.

6. A matrix according to claim 1 wherein the ester compound of a polyhydric alcohol with a fatty aliphatic acid is selected from the group consisting of esters obtained from (i) a glycerol or glycol component and (ii) a fatty aliphatic acid component having from 8 to 18 carbon atoms.

7. A matrix according to claim 6 wherein the glycol component is selected from the group consisting of ethylene glycol propylene glycol, dipropylene glycol, butylene glycol, triethylene glycol, diethylene glycol, polyethyleneglycol and polypropyleneglycol.

8. A matrix according to claim 6 wherein the fatty aliphatic acid component is selected from the group consisting of pelargonic acid, stearic acid, isostearic acid, capric acid, caprylic acid, palmitic acid, lauric acid, myristic acid and oleic acid.

9. A matrix according to claim 1 wherein the active ingredient to be administered percutaneously is an anti-inflammatory agent, in particular ibuprofen, ketoprofen, niflumic acid or mefenamic acid.

10. A matrix according to claim 1 wherein the active ingredient to be administered percutaneously is a calcic antagonist of the dihydropyridine type, such as nifedipine or nicardipine.

11. A matrix according to claim 1 wherein the active ingredient to be administered percutaneously is a $\beta$-blocking agent, such as timolol or propranolol.

12. A matrix according to claim 1 wherein the active ingredient to be administered percutaneously is a $\beta$-stimulating agent, such as procaterol or salbutamol.

13. A matrix according to claim 1 wherein the weight ratio $(a + c)/(b + d)$ is about 1.

14. A method for preparing a matrix as claimed in claim 1, coated on a support, said method comprising the following steps :

1) the means a) and part of the means b) are mixed, with stirring, at a temperature greater than or equal to 110°C, and the resulting mixture is homogenized for 0.5h,

2) the means c) is incorporated per small portions into the homogenized mixture resulting from stage 1, with stirring, at a temperature greater than or equal to 110°C, then the resulting mixture is homogenized,

3) the remainder of the means b) is incorporated into the homogenized mixture resulting from stage 2, with stirring, at a temperature greater than or equal to 110°C,

4) the resulting mixture thus obtained is homogenized at a temperature greater than or equal to 110°C and then left to stand for at least 8 hours,

5) the homogenized resulting mixture thus obtained is heated at a temperature of 50-70°C for at least 0.25 h, after which the means d) and the active ingredient in a solvent for the said active ingredient are incorporated at said temperature of 50-70°C, said solvent representing from 30 to 100 % v/w with respect to the total weight of means a), b), c), d) and e),

6) the resulting mixture thus obtained is homogenized for at least 0.5 h at a temperature of 50-70°C,

7) the homogenized resulting mixture thus obtained is deposited on a temporary support, at a temperature of 50-70°C, at a rate of 100 to 300 g/m$^2$,

8) the whole assembly consisting of said temporary support and said matrix is heated at a temperature of the order of 70-90°C in order to evaporte the solvent for the active ingredient until the residual proportion is less than 5 %, and

9) the resulting dry matrix is transferred onto an appropriate support.

**Claims for the following Contracting States : ES, GR,**

1. A method for preparing a self-adhesive matrix for the percutaneous administering of a pharmaceutical active ingredient and comprising

   a) 30 to 50 parts by weight of an ethylene-vinyl acetate copolymer material,

   b) 20 to 45 parts by weight of a higher aliphatic monoalcohol compound, selected from the $C_{12}$-$C_{20}$ fatty monoalcohol,

   c) 5 to 20 parts by weight of a cellulose derivative material,

   d) 1 to 20 parts by weight of an ester compound of a polyhydric alcohol with a fatty aliphatic acid, and

   e) 0.1 to 20 parts by weight of an active ingredient which can be administered percutaneously,

   said method comprising the following steps :

   1) the means a) and part of the means b) are mixed, with stirring, at a temperature greater than or equal to 110°C, and the resulting mixture is homogenized for 0.5h,

   2) the means c) is incorporated per small portions into the homogenized mixture resulting from stage 1, with stirring, at a temperature greater than or equal to 110°C, then the resulting mixture is homogenized,

   3) the remainder of the means b) is incorporated into the homogenized mixture resulting from stage 2, with stirring, at a temperature greater than or equal to 110°C,

   4) the resulting mixture thus obtained is homogenized at a temperature greater than or equal to 110°C and then left to stand for at least 8 hours,

   5) the homogenized resulting mixture thus obtained is heated at a temperature of 50-70°C for at least 0.25 h, after which the means d) and the active ingredient in a solvent for the said active ingredient are incorporated at said temperature of 50-70°C, said solvent representing from 30 to 100 % v/w with respect to the total weight of means a), b), c), d) and e),

   6) the resulting mixture thus obtained is homogenized for at least 0.5 h at a temperature of 50-70°C,

   7) the homogenized resulting mixture thus obtained is deposited on a temporary support, at a temperature of 50-70°C, at a rate of 100 to 300 g/m², 

   8) the whole assembly consisting of said temporary support and said matrix is heated at a temperature of the order of 70-90°C in order to evaporte the solvent for the active ingredient until the residual proportion is less than 5 %, and

   9) the resulting dry matrix is transferred onto an appropriate support.

2. A method according to claim 1 wherein the ethylene/vinyl acetate copolymer material has a content of vinyl acetate units of between 35 to 55 % by weight, relative to the weight of said copolymer material.

3. A method according to claim 1 wherein the ethylene/vinyl acetate copolymer material has a content of vinyl acetate units of 45 % by weight, relative to the weight of said copolymer material.

4. A method according to claim 1 wherein the $C_{12}$-$C_{20}$ higher aliphatic monoalcohol compound is selected from the group consisting of saturated and unsaturated monoalcohols.

5. A method according to claim 1 wherein the cellulose derivative material is selected from the group consisting of alkyl celluloses and hydroxyalkyl celluloses, in particular methyl cellulose, ethyl cellulose, propyl cellulose, methylpropyl cellulose, hydroxymethyl cellulose, hydroxyethyl cellulose and hydroxypropyl cellulose.

6. A method according to claim 1 wherein the ester compound of a polyhydric alcohol with a fatty aliphatic acid is selected from the group consisting of esters obtained from (i) a glycerol or glycol component and (ii) a fatty aliphatic acid component having from 8 to 18 carbon atoms.

7. A method according to claim 6 wherein the glycol component is selected from the group consisting of ethylene glycol propylene glycol, dipropylene glycol, butylene glycol, triethylene glycol, diethylene glycol, polyethyleneglycol and polypropyleneglycol.

8. A method according to claim 6 wherein the fatty aliphatic acid component is selected from the group consisting of pelargonic acid, stearic acid, isostearic acid, capric acid, caprylic acid, palmitic acid, lauric acid, myristic acid and oleic acid.

9. A method according to claim 1 wherein the active ingredient to be administered percutaneously is an anti-inflammatory agent, in particular ibuprofen, ketoprofen, niflumic acid or mefenamic acid.

10. A method according to claim 1 wherein the active ingredient to be administered percutaneously is a calcic antagonist of the dihydropyridine type, such as nifedipine or nicardipine.

11. A method according to claim 1 wherein the active ingredient to be administered percutaneously is a β-blocking agent, such as timolol or propranolol.

12. A method according to claim 1 wherein the active ingredient to be administered percutaneously is a β-stimulating agent, such as procaterol or salbutamol.

13. A method according to claim 1 wherein the weight ratio $(a+c)/(b+d)$ is about 1.

**Patentansprüche**
**Patentansprüche für folgende Vertragsstaaten : AT, BE, CH, DE, FR, GB, IT, LI, LU, NL, SE**

1. Selbstklebende Matrix zur Verabreichung eines Wirkstoffes auf perkutanem Weg, dadurch gekennzeichnet, daß sie umfaßt:
   (a) 30 bis 50 Gewichtsteile eines Copolymers von Ethylen und Vinylacetat,
   (b) 20 bis 45 Gewichtsteile eines höheren aliphatischen Alkohols ausgewählt unter den $C_{12}$-$C_{20}$ -Monofettalkoholen,
   (c) 5 bis 20 Gewichtsteile eines Cellulosederivates,
   (d) 1 bis 20 Gewichtsteile eines Esters einer Fettsäure und eines mehrwertigen Alkohols, und
   (e) 0,1 bis 20 Gewichtsteile des auf perkutanem Weg verabreichbaren Wirkstoffes.

2. Matrix nach Anspruch 1, dadurch gekennzeichnet, daß das Copolymer aus Ethylen und Vinylacetat einen Gehalt an Vinylacetateinheiten zwischen 35 und 55 Gewichtsprozent, bezogen auf das Gewicht des Copolymers, aufweist.

3. Matrix nach Anspruch 2, dadurch gekennzeichnet, daß das Copolymer aus Ethylen und Vinylacetat einen Gehalt an Vinylacetateinheiten von 45 Gewichtsprozent, bezogen auf das Gewicht des Copolymers, aufweist.

4. Matrix nach Anspruch 1, dadurch gekennzeichnet, daß der höhere aliphatische $C_{12}$-$C_{20}$-Alkohol ausgewählt ist unter der Gruppe bestehend aus gesättigten und ungesättigten Monoalkoholen.

5. Matrix nach Anspruch 1, dadurch gekennzeichnet, daß das Cellulosederivat ausgewählt ist aus der Gruppe bestehend aus den Alkylcellulosen und Hydroxyalkylcellulosen, insbesondere Methylcellulose, Ethylcellulose, Propylcellulose, Methylpropylcellulose, Hydroxymethylcellulose, Hydroxyethylcellulose und Hydroxypropylcellulose.

6. Matrix nach Anspruch 1, dadurch gekennzeichnet, daß der Ester der Fettsäure und des mehrwertigen Alkohols ausgewählt ist aus der Gruppe bestehend aus den aus Glycerin oder einem Glykol und einer Säure mit einer Gesamtzahl von 8 bis 18 Kohlenstoffatomen erhaltenen Estern.

7. Matrix nach Anspruch 6, dadurch gekennzeichnet, daß das Glykol ausgewählt ist unter der Gruppe bestehend aus den Alkylenglykolen, insbesondere Ethylenglykol, Propylenglykol, Dipropylenglykol, Butylenglykol, Triethylenglykol, Diethylenglykol, Polyethylenglykol, Polypropylenglykol.

8. Matrix nach Anspruch 6, dadurch gekennzeichnet daß die Fettsäure ausgewählt ist aus der Gruppe bestehend aus Pelargonsäure, Stearinsäure, Isostearinsäure, Caprinsäure, Caprylsäure, Palmitinsäure, Laurinsäure, Myristinsäure, Ölsäure.

9. Matrix nach Anspruch 1, dadurch gekennzeichnet, daß der auf perkutanem Weg verabreichbare Wirkstoff ein nichtsteroides anti-inflammatorisches Mittel, insbesondere Ibuprofen, Ketoprofen, Nifluminsäure oder Mefenaminsäure ist.

10. Matrix nach Anspruch 1, dadurch gekennzeichnet, daß der auf perkutanem Weg verabreichbare Wirkstoff ein Calcium-Antagonist vom Typ Dihydropyridin, insbesondere das Nifedipin oder das Nicardipin, ist.

11. Matrix nach Anspruch 1, dadurch gekennzeichnet, daß der auf perkutantem Weg verabreichbare Wirkstoff ein β-Blocker, insbesondere Timolol oder Propranolol, ist.

12. Matrix nach Anspruch 1, dadurch gekennzeichnet, daß der auf perkutanem Weg verabreichbare Wirkstoff ein β-Stimulans, insbesondere Procaterol oder Salbutamol, ist.

**13.** Matrix nach Anspruch 1, dadurch gekennzeichnet, daß das Gewichtsverhältnis (a) + (c) / (b) + (d) in der Größenordnung von 1 ist.

**14.** Verfahren der Herstellung einer Matrix nach Anspruch 1, dadurch gekennzeichnet, daß man:

(1) die Komponente (a) und einen Teil der Komponente (b) bei einer Temperatur oberhalb oder gleich 110 °C unter Rühren mischt und die Mischung während 0,5 Stunden homogenisiert,

(2) unter Rühren in kleinen Anteilen bei einer Temperatur oberhalb oder gleich 110 °C die Komponente (c) in die resultierende Mischung der Stufe (1) zu gibt und schließlich homogenisiert,

(3) den Rest der Komponente (b) unter Rühren bei einer Temperatur oberhalb oder gleich 110 °C in die aus Stufe (2) resultierende Mischung einbringt,

(4) die so erhaltene Mischung bei einer Temperatur oberhalb oder gleich 110 °C homogenisiert und schließlich mindestens 8 Stunden beläßt,

(5) die so erhaltene Mischung bei einer Temperatur von 50-70 °C während mindestens 0,25 Stunden erhitzt und schließlich bei dieser Temperatur die Komponente (d) und den Wirkstoff in einem Lösungsmittel für den Wirkstoff, wobei das Lösungsmittel 30 bis 100 Volumenprozent bezogen auf das Gesamtgewicht der Bestandteile (a), (b), (c), (d) und (e) beträgt, einbringt,

(6) die resultierende Mischung während mindestens 0,5 Stunden bei einer Temperatur von 50-70 °C homogenisiert,

(7) die so homogenisierte resultierende Mischung auf einen temporären Träger, insbesondere Silikonpapier, bei einer Temperatur von 50-70 °C in einer Menge von 100 bis 300 g/m² aufbringt,

(8) den temporären Träger und die Matrix bei einer Temperatur von 70-90 °C erhitzt, um das Lösungsmittel für den Wirkstoff bis zu einem Restgehalt von weniger als 5 % verdampfen,

(9) die resultierende trockene Matrix auf einen geeigneten Träger überträgt.

**Patentansprüche für folgende Vertragsstaaten : ES, GR**

**1.** Verfahren zur Herstellung einer selbstklebenden Matrix zur Verabreichung eines Wirkstoffes auf perkutanem Weg, die umfasst:

(a) 30 bis 50 Gewichtsteile eines Copolymers aus Ethylen und Vinylacetat,

(b) 20 bis 45 Gewichtsteile eines höheren aliphatischen Alkohols ausgewählt aus den $C_{12}$-$C_{20}$ -Fettmonoalkoholen,

(c) 5 bis 20 Gewichtsteile eines Cellulosederivates,

(d) 1 bis 20 Gewichtsteile eines Esters einer Fettsäure und eines mehrwertigen Alkohols, und

(e) 0,1 bis 20 Gewichtsteile des auf perkutanem Weg verabreichbaren Wirkstoffes,

dadurch gekennzeichnet, daß man

(1) die Komponente (a) und einen Teil der Komponente (b) bei einer Temperatur oberhalb oder gleich 110 °C unter Rühren mischt und die Mischung während 0,5 Stunden homogenisiert,

(2) unter Rühren in kleinen Anteilen bei einer Temperatur oberhalb oder gleich 110 °C die Komponente (c) in die resultierende Mischung der Stufe (1) zu gibt und schließlich homogenisiert,

(3) den Rest der Komponente (b) unter Rühren bei einer Temperatur oberhalb oder gleich 110 °C in die aus Stufe (2) resultierende Mischung einbringt,

(4) die so erhaltene Mischung bei einer Temperatur oberhalb oder gleich 110 °C homogenisiert und schließlich mindestens 8 Stunden beläßt,

(5) die so erhaltene Mischung bei einer Temperatur von 50-70 °C während mindestens 0,25 Stunden erhitzt und schließlich bei dieser Temperatur die Komponente (d) und den Wirkstoff in einem Lösungsmittel für den Wirkstoff, wobei das Lösungsmittel 30 bis 100 Volumenprozent bezogen auf das Gesamtgewicht der Bestandteile (a), (b), (c), (d) und (e) beträgt, einbringt,

(6) die resultierende Mischung während mindestens 0,5 Stunden bei einer Temperatur von 50-70 °C homogenisiert,

(7) die so homogenisierte resultierende Mischung auf einen temporären Träger bei einer Temperatur von 50-70 °C in einer Menge von 100 bis 300 g/m² aufbringt,

(8) den temporären Träger und die Matrix bei einer Temperatur von 70-90 °C erhitzt, um das Lösungsmittel für den Wirkstoff bis zu einem Restgehalt von weniger als 5 % verdampfen,

(9) die resultierende trockene Matrix auf einen geeigneten Träger überträgt.

**2.** Verfahren nach Anspruch 1, dadurch gekennzeichnet, daß das Copolymer aus Ethylen und Vinylacetat einen Gehalt an Vinylacetateinheiten zwischen 35 und 55 Gewichtsprozent, bezogen auf das Gewicht des Copolymers, aufweist.

3. Verfahren nach Anspruch 2, dadurch gekennzeichnet, daß das Copolymer aus Ethylen und Vinylacetat einen Gehalt an Vinylacetateinheiten von 45 Gewichtsprozent, bezogen auf das Gewicht des Copolymers, aufweist.

4. Verfahren nach Anspruch 1, dadurch gekennzeichnet, daß der höhere aliphatische Alkohol ausgewählt ist unter der Gruppe bestehend aus gesättigten und ungesättigten $C_{12}$-$C_{20}$-Monoalkoholen.

5. Verfahren nach Anspruch 1, dadurch gekennzeichnet, daß das Cellulosederivat ausgewählt ist aus der Gruppe bestehend aus Alkylcellulosen und Hydroxyalkylcellulosen, insbesondere Methylcellulose, Ethylcellulose, Propylcellulose, Methylpropylcellulose, Hydroxymethylcellulose, Hydroxyethylcellulose und Hydroxypropylcellulose.

6. Verfahren nach Anspruch 1, dadurch gekennzeichnet, daß der Ester einer Fettsäure und eines mehrwertigen Alkohols ausgewählt ist aus der Gruppe bestehend aus den aus Glycerin oder einem Glykol und eine Säure mit einer Gesamtzahl von 8 bis 18 Kohlenstoffatomen erhaltenen Estern.

7. Verfahren nach Anspruch 6, dadurch gekennzeichnet, daß das Glykol ausgewählt ist aus der Gruppe bestehend aus den Alkylenglykolen, insbesondere Ethylenglykol, Propylenglykol, Dipropylenglykol, Butylenglykol, Triethylenglykol, Diethylenglykol, Polyethylenglykol, Polypropylenglykol.

8. Verfahren nach Anspruch 6, dadurch gekennzeichnet, daß die Fettsäure ausgewählt ist aus der Gruppe bestehend aus Pelargonsäure, Stearinsäure, Isostearinsäure, Caprinsäure, Caprylsäure, Palmitinsäure, Laurinsäure, Myristinsäure, Ölsäure.

9. Verfahren nach Anspruch 1, dadurch gekennzeichnet, daß der auf perkutanem Weg verabreichbare Wirkstoff ein nicht-steroides anti-inflammatorisches Mittel, insbesondere Ibuprofen, Ketoprofen, Nifluminsäure oder Mefenaminsäure, ist.

10. Verfahren nach Anspruch 1, dadurch gekennzeichnet, daß der auf perkutanem Weg verabreichbare Wirkstoff ein Calcium-Antagonist vom Typ Hydroxypyridin, insbesondere das Nifedipin oder das Nicardipin, ist.

11. Verfahren nach Anspruch 1, dadurch gekennzeichnet, daß der auf perkutanem Weg verabreichbare Wirkstoff ein β-Blocker, insbesondere Timolol oder Propranolol ist.

12. Verfahren nach Anspruch 1, dadurch gekennzeichnet, daß der auf perkutanem Weg verabreichbare Wirkstoff ein β-stimulierendes Mittel, insbesondere Procaterol oder Salbutamol, ist.

13. Verfahren nach Anspruch 1, dadurch gekennzeichnet, daß das Gewichtverhältnis (a) + (c) / (b) + (d) in der Größenordnung von 1 ist.